# EUROPÄISCHE PATENTSCHRIFT

(11) **EP 0 454 044 B1**
(45) Veröffentlichungstag und Bekanntmachung des Hinweises auf die Patenterteilung: **06.12.1995**
(21) Anmeldenummer: 91106495.4
(22) Anmeldetag: 23.04.1991
(51) Int. Cl.: A61K 9/14, A61K 9/16, A61K 9/50

(54) **Pharmakologische Zubereitung, enthaltend Polyelektrolytkomplexe in mikropartikulärer Form und mindestens einen Wirkstoff**
Pharmacological product containing polyelectrolyte complexes in microparticulate form and at least one substance
Produit pharmacologique contenant des composés de polyélectrolytes sous forme de microparticules et au moins un agent actif

(30) Priorität: 25.04.1990 DE 4013110; 06.11.1990 DE 4035187
(43) Veröffentlichungstag der Anmeldung: 30.10.1991
(73) Patentinhaber: HOECHST AKTIENGESELLSCHAFT, 65926 Frankfurt am Main (DE)
(72) Erfinder: Krone, Volker, Dr., W-6238 Hofheim am Taunus (DE); Magerstädt, Michael, Dr., W-6238 Hofheim am Taunus (DE); Walch, Axel, Dr., W-6000 Frankfurt am Main 90 (DE); Gröner, Albrecht, Dr., W-6104 Seeheim-Jugenheim (DE); Hoffmann, Dieter, Dr., W-3550 Marburg (DE)

(56) Entgegenhaltungen:
- EP-A- 0 152 898
- EP-A- 0 388 758
- EP-A- 0 392 487
- GB-A- 1 183 403
- GB-A- 2 153 780

## Beschreibung

Die Erfindung betrifft pharmazeutische Zubereitungen, in mikropartikulärer Form enthaltend einen Polyelektrolytkomplex aus Polykationen und Polyanionen und mindestens einen Wirkstoff. Der Wirkstoff kann dabei in die Matrix des Polyelektrolytkomplexes eingebettet sein, selbst ein Partner in dem Polyelektrolytkomplex sein oder an einem Partner des Polyelektrolytkomplexes gebunden sein. Unter Wirkstoffen sind in erster Linie pharmakologische Wirkstoffe zu verstehen, wie aktive Peptide, Proteine, Enzyme, Enzyminhibitoren, Antigene, Cytostatika, Antibiotika, Antiinflammatorika oder Vakzine. In dem besonderen Fall der Utraschalldiagnostika sollen hier auch konstrastgebende Mittel wie Gase, z.B. Luft, Sauerstoff oder Edelgase als Wirkstoffe verstanden werden.

Es ist literaturbekannt, daß mehrfach geladene makromolekulare Verbindungen mit Ionen entgegengesetzter Ladung ionische Verbindungen bilden, die, abhängig von der Ladungsverteilung und dem Molekulargewicht des Endproduktes, aus wäßrigen Lösungen ausfallen können. Dabei werden niedermolekulare Ionen gleicher Ladung durch die höhermolekulare Verbindung verdrängt. Diese Phänomene werden auch unter dem Oberbegriff "Polyelektrolyteffekt" zusammengefaßt. Stand der Technik sind u.A. die Ausbildung von Gelen durch zusammengeben von Alginatlösungen und Ca²⁺. Auch Proteinfällungen laufen z.T. nach diesem Prinzip ab.
Polyelektrolythkomplexe können prinzipiell entweder aus einer makromolekularen, mehrfach geladenen Komponente einer Polarität und vielen niedermolekularen Ionen der anderen Polarität, oder aber aus zwei makromolekularen, jeweils mehrfach geladenen Partnern verschiedener Polarität bestehen. Hohlkapseln, die aus solchen Polyelektrolytkomplexen hergestellt werden, sind beispielsweise beschrieben in BE-A 901.704, EP-A 0 127 989, EP-A 0 188 309, EP-A 0 127 713, DE-A 32 09 127, EP-A 0 152 898, US 4,487,758, DE-A 32 09 098, US 4,409,331, A. Johansen und J.M. Flink, Enzyme Mikrob. Technol., 1986, vol.8, 145-148 oder C.E. Camp und S.S. Sofer, Enzyme Mikrob. Technol., 1987, vol.9, 685-689.

In EP-A 0 392 487 werden Anthracyclin-Polyanionen-Komplexe beschrieben, also Komplexe, die nur eine polyionische Verbindung enthalten. Sie stellen keine Mikropartikel definierter Größe dar. In EP-A-0 388 758 wird ein System für den Transport von Nukleinsäuren in die Zelle, welcher über Rezeptor vermittelte Endozytose abläuft, beschrieben. Dabei wird ein Polykation-Konjugat mit der polyanionischen Nuklensäure komplexiert. Es bilden sich vollständig lösliche Komplexe, aber keine Mikropartikel. GB-A-1 183 403 beschreibt ein Anti-Husten Medikament, welches ein sulfatiertes Polysaccharid und ein Opium-Alkaloid enthält. Die Herstellung erfolgt bei 40 - 60°C. Mikropartikel werden nicht beschrieben.

In der modernen pharmazeutischen Technologie werden Formulierungen und Wirkstoffkombinationen immer wichtiger, die nicht nur den Wirkstoff auf schonende Weise in eine applizierbare Form bringen, sondern die gezielt die Bioverteilung, Bioverfügbarkeit oder Resorption des Arzneimittels beeinflussen. Hiermit sind sowohl neue therapeutische und diagnostische Anwendungsgebiete erreichbar als auch z.B. eine Erhöhung der therapeutischen Breite eines Wirkstoffs. Insbesondere partikuläre Systeme kleinsten Durchmessers (sogenannte Mikro- bzw. Nanopartikel) deuten sich in jüngster Zeit als wichtige Applikationsform sowohl im oralen als auch im parenteralen Bereich an. Hierbei kommen meist biokompatible bioabbaubare Polymere als Trägersubstanzen zum Einsatz.

Überraschend wurde nun gefunden, daß Polyelektrolytkomplexe in besonderem Maße sowohl als Trägersubstanzen als auch als Wirkstoffkomponenten Eigenschaften zeigen, die dem Anforderungsprofil biokompatibler (bioabbaubarer) Polymersysteme entsprechen und die verschiedenen Anforderungen angepaßt werden können. Von Hohlkapseln, auch Polyelektrolytkomplexkapseln her war bekannt, daß Wirkstoffeinschlußkapazitäten von 90 % und mehr erreicht werden können. Dies war bei Mikropartikeln, die aus ebensolchen Polyelektrolytkomplexen aufgebaut werden, nicht zu erwarten, da sich diese Komplexe nur an der Grenzfläche (um den Wirkstoff herum) bilden. Dies gilt insbesondere, wenn sie aus rein wäßrigen Reaktionslösungen hergestellt werden, in die darin lösliche Wirkstoffe eingebracht sind. Es war deshalb um so überraschender, daß auch partikuläre Polyelektrolytkomplexe Einschlußkapazitäten von über 90 % aufwiesen. Es war ferner überraschend, daß sich bei der Herstellung solcher Polyelektrolytkomplexmatrizes feine Partikel im »m-Bereich oder Emulsionen bildeten und nicht - wie eigentlich zu erwarten war - eine verklumpte Masse. Erfindungsgemäß lassen sich solche kolloidalen Systeme von Mikro/Nanopartikeln besonders gut aus Polyelektrolytkomplexen herstellen. Über Lösungsgleichgewichte und Ladungswechselwirkungen erfolgt dann in vivo ein langsames Dekomplexieren und auch ein Abbau der Komplexanden, was zur Auflösung des Komplexes unter Wirkstofffreigabe führt. Diese Freigabebedingungen können ebenso wie die Konsistenzeigenschaften über die Komplexzusammensetzung gesteuert werden. Als Partner zur Komplexbildung werden bevorzugt natürlich vorkommende oder aus natürlichen Bausteinen bestehende biokompatible bioabbaubare Polysäuren und Polybasen eingesetzt. "Poly" bedeutet in diesem Fall, daß die Verbindung mehr als eine Ladung derselben Polarität, bevorzugt eine große Zahl solcher Ladungen, trägt. Die jeweiligen Gegenionen können entweder aus niedermolekularen Ionen oder ebenfalls aus einer polyionischen Spezies bestehen. Sowohl einer, als auch beide ionischen Partner können sowohl anorganischer als auch organischer Natur sein. Im Falle organischer Polyionen erweisen sich vorzugsweise hydrophobe substituierte Derivate als geeignet.

Bevorzugte Materialien für die Herstellung biokompatibler Polyelektrolytkomplex-Wirkstoff-Kombinationen sind als Polysäuren: Xylanpolysulfat, teilweise hydrophob verestertes Xylanpolysulfat, Polysulfate anderer Polyzucker wie z.B. Stärkehydrolysate, Inulin, Hydroxyethylstärke, Dextrane und deren jeweils teilweise hydrophob substituierte Derivate, sowie Polyaminosäuren wie Polyasparaginsäure oder Polyglutaminsäureund denen jeweils hydrophob substituierte Derivate. Als Polybasen: Poly-L-Lysin verschiedener definierter Molekulargewichtsbereiche, Poly-α,β-(2-dimethylamino ethyl)-D,L-aspartamid (PDAA), Copolymere aus PDAA und hydrophob verestertem Poly-α,β-(2-hydroxyethyl)-D,L-aspartamid (PHEA), Chitosan, Lysinoctadecylester, aminierte Dextrane, aminierte Cyclodextrine, aminierte Celluloseether, aminierte Pektine sowie deren jeweils teilweise hydrophob substituierte Derivate.

Mikropartikel aus Polyelektrolytkomplexen können je nach Anforderungen in Durchschnittsteilchengrößen von einigen nm bis zu einigen 100 »m hergestellt werden. Auch können die Mikropartikel definitionsgemäß als Emulsionen vorliegen. Die Breite der Größenverteilung ist einstellbar, beispielsweise über die Rührgeschwindigkeit beim Zusammengeben der Polyelektrolyte, die Tropfgeschwindigkeit, den Düsendurchmesser, den pH-Wert und durch geeignete Auswahl der Polyelektrolytpartner. Besonders vorteilhaft ist, daß die Bildung der Komplexe auch bei Zugabe von Hilfsstoffen wie amphiphilen Molekülen (z.D. ®Pluronic) oder kolloidalen Substanzen (z.B. Adjuvantien) mit hoher Einschlußkapazität erfolgt. Diese Parameter können in einfachen Routineversuchen ermittelt und& an aie gewünschte Teilchengröße und Teilchengrößenverteilung angepaßt werden. Teilchen von unter 5 »m Durchmesser eignen sich zur intravenösen Injektion. Teilchen mit einem Durchmesser <15 »m, bevorzugt <10 »m können als s.c. oder i.m. injizierbare Depotformen sowie als Vehikel zur Erhöhung der enteralen Resorption eingesetzt werden.

Der Einschluß eines Wirkstoffs in Polyelektrolytkomplexpartikel/-kolloide kann auf zumindest 4 Wegen erfolgen: a) Einschluß durch "Einfangen" des in der Lösung befindlichen Wirkstoffs bei der Komplexfällung, b) Einschluß durch Absorption des Wirkstoffs aus einer Lösung, mit der die bereits hergestellten Polyelektrolytkomplexe in Kontakt kommen (besonders bei porösen Materialien oder Gelen mit "Schwamm"-Eigenschaften), c) Ausfällung des Polyelektrolytkomplexes, wobei der Wirkstoff chemisch an mindestens einen Komplexpartner gebunden ist, d) Einschluß durch Einsatz des Wirkstoffs als Polyelektrolytkomplexbildungspartner. Dies erfordert zumeist mindestens eine Ladung oder polarisierbare Gruppe am Wirkstoff.

Die Erfindung betrifft deshalb auch ein Verfahren zur Herstellung von Polyelektrolytkomplexe und Wirkstoffe enthaltenden pharmazeutischen Zubereitungen, wobei eine Lösung von einer sauren und eine Lösung von einer basischen Substanz, wobei mindestens eine dieser Substanzen polymer sein muß, zusammengegeben werden und wobei a) entweder einer der Partner ein Wirkstoff ist oder diesen in chemisch gebundener Form enthält oder b) der Wirkstoff in einer der Lösungen enthalten ist und anschließend der entstandene Polyelektrolytkomplex in mikropartikulärer Form ausfällt oder gegebenenfalls in eine mikropartikuläre Form überführt wird.

Wegen der einerseits weit variablen, andererseits sehr spezifisch einstellbaren Konsistenzeigenschaften zeigen Polyelektrolythkomplex-Wirkstoff-Formulierungen Eigenschaftsprofile, wie sie für unterschiedliche pharmazeutische Anwendungen gewünscht werden.

So zeigte es sich, daß aus dem Cytostatikum Daunorubicin und der Polysäure Xylanpolysulfat Mikropartikel entstehen, die Daunorubicin enthalten und dieses in Pufferlösung bzw. in biologischen Systemen über einen längeren Zeitraum gleichmäßig freisetzen, wobei sie abgebaut werden. Setzt man noch Polybasen zu oder/und variiert man die Polysäure - insbesondere durch Ersatz von Xylanpolysulfat durch teilweise mit Palmitoylestergruppen substituiertes Xylanpolysulfat - so kann die Partikelgröße auf <<5 »m gesenkt werden und man erhält ein i.v. injizierbares System mit den oben beschriebenen Freisetzungseigenschaften. Mit einer solchen slow-release-Form kann die therapeutische Breite des Cytostatikums drastisch erhöht werden. Auch andere niedermolekulare Wirkstoffe wie Antibiotika (z.B. Tetracyclin) oder andere Cytostatika können so in ihren Wirkeigenschaften deutlich verbessert werden.

Schließt man Proteine in Polyelektrolytkomplexmikropartikel ein, kann man diese dadurch sowohl vor hydrolytischen Angriffen schützen als auch kontrollierte Freisetzungsprofile erreichen. So kann man z.B. mit Virusproteinen oder ähnlichen zur Vakzinierung geeigneten Substanzen Impfstoffpräparationen erzeugen, die, je nach Partikelgröße, i.m. injizierbar oder sogar oral applizierbar sind, wobei bei Partikeln <5 »m Resorption im Gastrointestinaltrakt erfolgt und subsequent Antigenexpression/Immunisierung auftritt. Mit solchen erfindungsgemäßen Antigen enthaltenden Polyelektrolytkomplexen ist es möglich, Releaseprofile zu erreichen, die ein stoßweises Abgeben des Impfstoffes kurz nach der Applikation sowie nach einem Zeitraum von z.B. 4 Wochen (Booster) ermöglichen. Die in diesem Fall besonders zur Polyelektrolytkomplexbildung geeigneten Substanzen sind in Beispiel 3 beschrieben.

Auch Wirkstoffe auf Peptidbasis können durch Polyelektrolytkomplexpräparationen in geeignete Langzeitsysteme überführt werden. Diese Formulierungen sind z.T. den bekannten polymeren Depotsystemen für z.B. LHRH-Analoga sowohl aufgrund besserer Abbaubarkeit als auch aufgrund definierterer Freisetzungsprofile überlegen.

Ebenso eignen sich Polyelektrolytkomplexe zur Herstellung von Wundsalbenpräparationen, die z.B. Antibiotika oder Proteine als Regenerationsförderer enthalten.

Auch als Luft enthaltende echogene Kontrastmittel für die Ultraschalldiagnostik sind die erfindungsgemäßen Polyelektrolytkomplexmikropartikel hervorragend geeignet. Als besonders geeignet für die Ultraschalldiagnostik erwiesen sich Polyelektrolytkomplexpartikel aus hydrophob verestertem Dextransulfat und einem Copolymer aus PDAA und hydrophob verestertem PHEA (Abkürzungen siehe Seite 4).

Im Folgenden ist die Erfindung anhand von Beispielen näher erläutert. Die Partikelgröße ist über mikroskopische Methoden oder mittels Filtration durch Filter definierter Porengröße sowie z.T. über Coulter-Counter (Fa. Coulter Electronics) bzw. Durchflußcytometer bestimmt worden.

### Beispiele

### a) Präparation von Polyelektrolytkomplexen

### Beispiel 1

### Komplex aus Xylanpolysulfat und Poly-L-Lysin 3800

Je eine 0.1% wäßrige Lösung von Xylanpolysulfat-Natriumsalz (Fa. BENE-Chemie) und Poly-L-Lysin des mittleren Molekulargewichts 3800 (Fa. Sigma) wird angesetzt. Zur Poly-L-Lysinlösung wird so viel HCl gegeben, daß der pH bei 3 liegt. Die Xylanpolysulfatlösung wird ebenfalls auf pH 3 gebracht (HCl) und über eine Dosierpipette zugetropft. Der Polyelektrolytkomplex fällt aus und wird durch Zentrifugieren und Membranfiltration abgetrennt. Nach Waschen mit H₂0 kann das mikropartikuläre Produkt gefriergetrocknet werden. Die Partikelgröße läßt sich durch Gefäßgröße, Rührgeschwindigkeit, Zutropfdüsendurchmesser und Tropfgeschwindigkeit steuern und ist vom Bereich um 20 nm bis 100 »m einstellbar.

### Beispiel 2

### Komplex aus Palmitoylxylanpolysulfat mit 20% Palmitinsäureresten und Chitosan

Wie in Beispiel 1 werden 0.1 % Lösungen hergestellt. Die Prozedur entspricht in der Durchführung Beispiel 1, nur daß hier keine pH-Regulierung erfolgt und Polylysin durch Chitosan (Fa. Protan) ersetzt wird. Das Palmitoylxylanpolysulfat kann beispielsweise nach dem in der deutschen Patentanmeldung P 3921761.2 beschriebenen Verfahren hergestellt werden. Chitosan 143 wird verwendet. Die erhaltenen Teilchen sind große Agglomerate (100 »m und größer) und können durch Zerreiben im Mörser auf 1-4 »m Größe zerkleinert werden.

### Beispiel 3

### Polyelektrolytkomplexpartikel aus Palmitoylxylanpolysulfat mit 20% Palmitinsäure und Chitosan unter Einschluß von Humanserumalbumin als Modellprotein für Vakzine

Wie in Beispiel 2 beschrieben wird verfahren, nur daß der Palmitoylxylanpolysulfatlösung vor dem Zutropfen 0.2 % Humanserumalbumin (Fa. Sigma), in Wasser gelöst, zugesetzt werden.
Nach Zerreiben können Partikel im Bereich von 2-5 »m erhalten werden. Zur Bestimmung der Albuminfreisetzung siehe Beispiel 10.

### Beispiel 4

### Herstellung von Tollwutvakzin-Polyelektrolytkomplex-Mikropartikeln

Partikel im <5 »m Bereich können mit zwei verschiedenen Präparationen erhalten werden:
- I. Polysäure:: Palmitoylxylanpolysulfat mit 20 % Palmitinsäure
- Polybase:: Lysinoctadecylester
- Hilfsstoff:: ®Pluronic F68
50 mg Polysäure werden in 5 ml einer 0.1 %igen Lösung von Tollwutvakzin Behringwerke (die Lösung ist wäßrig und enthält 40 % Saccharose) gelöst, der pH beträgt 6.3. 50 mg Polybase werden in 5 ml einer 0.5 %igen Lösung von ®Pluronic F 68 in Wasser gegeben. Die Polysäure/Vakzin-Lösung wird unter Rühren zur Polybaselösung (welche pH 5.8 hat) getropft. Nach Zentrifugieren (10 Min., 2000 U/Min.) wird der klare Überstand abgetrennt, der Rückstand mit H₂O aufgeschlämmt und gefriergetrocknet. Ausbeute 779.7 mg Partikel. Durch Resuspension und Analyse des Überstandes (in H₂O) kann eine Einschlußmenge von 90 % des eingesetzten Vakzins gefunden werden.
- II. Polysäure:: Palmitoylxylanpolysulfat mit 20 % Palmitinsäure
- Polybase:: 40:60 Copolymer aus Poly-α,β-(2-dimethylaminoethyl)-D,L-aspartamid (40 %) und Poly-α,β-(2-[Palmitoyl-oxy]ethyl)-D,L-aspartamid (60 %)
- kein Hilfsstoff -

Wieder werden zwei Lösungen mit jeweils 50 mg Polysäure/Base angesetzt. Die Polysäurelösung ist identisch zu der unter I. Die Polybaselösung ist der unter I identisch, außer, daß sie kein ®Pluronic enthält. Beide Lösungen werden auf pH 7 gestellt, wie unter I zentrifugiert, der Rückstand aufgeschlämmt und gefriergetrocknet. Die Einschlußeffizienz entspricht der unter I. Ausbeute: 76.5 mg.

### Beispiel 5

### Vakzinierung von Mäusen gegen Humanserumalbumin mit Polyelektrolytkomplex-Mikropartikeln

Folgende Mikropartikelpräparationen wurden eingesetzt:
- Probe I:: Xylansulfat, mit ca 15 % Palmitinsäure verestert/ Lysinoctadecylester + 7 % Pluronik^{R} F68, 5 - 30 »m
- Probe II:: Xylansulfat, mit ca. 15 % Palmitinsäure verestert/ Lysinoctadecylester, ≦10 »m
- Probe III:: Xylansulfat, mit ca. 15 % Palmitinsäüure verestert/ Poly-L-Lysin 4 k Da + 7 % Pluronic^{R} F68, 2 - 50 »m
- Probe IV:: Polyasparaginsäure 30 kDa/Poly-α,β-(2-dimethylamino ethyl)-D,L-aspartamid--Poly-α,β-(2-Palmitoyloxy)ethyl-D,L-aspartamid-Copolymer (40:60), <10 »m
- Probe V:: Xylansulfat/Lysinoctadecylester, 10 - 20 »m
Alle Proben enthielten ca. 7 Gew.-% Humanserumalbumin (Behringwerke). Diese Komplexe wurden in Konzentrationen von 66,67 »g/ml, 6,67 »g/ml bzw. 0,67 »g/ml in PBS (phosphatgepufferte Saline) resuspendiert. O,3 ml jeder Vakzine wurden je 10 NMRI-Mäusen, ca. 20 g schwer, s.c. appliziert. 14 Wochen post Vakzinierung wurden die Versuchstiere mit gleicher Dosis revakziniert. Die gegen Humanserumalbumin gerichteten Antikörper im Serum der Versuchstiere wurden im ELISA quantifiziert. Als Vergleich diente das als gutes Adjuvans bekannte und in verschiedenen Vakzinen enthaltene Aluminiumhydroxid Al(OH)₃.

ELISA-Titer nach Impfung mit 6,67 »g Formulierung/ml (mittlere Dosis), 2, 4, 8, 14 (Revakzinierung), 16 und 21 Wochen nach der ersten Vakzinierung:

| Probe | Tag 0 | Tag 2 | Tag 4 | Tag 8 | Tag 14 |
|---|---|---|---|---|---|
| I | <1:300 | <1:300 | 1:300 | 1:900 | 1:900 |
| II | <1:300 | <1:300 | 1:300 | <1:300 | <1:300 |
| III | <1:300 | 1:300 | 1:900 | 1:2700 | 1:8100 |
| IV | <1:300 | <1:300 | <1:300 | 1:2700 | 1:8100 |
| V | <1:300 | 1:900 | 1:2700 | 1:8100 | 1:24300 |
| Al(OH)₃ | <1:300 | 1:900 | 1:900 | 1:900 | 1:900 |

| Probe | Tag 16 | Tag 21 |
|---|---|---|
| I | 1:72900 | 1:72900 |
| II | 1:24300 | 1:24300 |
| III | 1:72900 | 1:24300 |
| IV | 1:72900 | 1:72900 |
| V | 1:72900 | 1:72900 |
| Al(OH)₃ | 1:8100 | 1:24300 |

Die Applikation derselben Vakzine in Meerschweinchen führte ebenfalls zu einer deutlichen Serokonversion.

### Beispiel 6

Polyelektrolytkomplexpartikel aus Polyasparaginsäure und Poly-α,β-(2-dimethylamino ethyl)-D,L-aspartamid (PDAA) unter Einschluß von Tetracyclin als Beispiel für einen niedermolekularen Wirkstoff.

Es wird wie in Beispiel 3 beschrieben verfahren, mit der Ausnahme, daß anstelle Humanserumalbumin eine 0.2 % Lösung von Tetracyclin in Wasser eingesetzt wird. Zur Tetracyclinfreisetzung siehe Beispiel 11.

### Beispiel 7

### Polyelektrolytkomplexpartikel aus Xylanpolysulfat und Daunorubicin

10 mg Xylanpolysulfat werden in 0.5 ml H₂0 gelöst. 100 »l einer 10% Daunorubicinlösung (Daunorubicin von Fa. Sigma) werden mit Wasser auf 0.4 ml verdünnt. Die Daunorubicinlösung wird zu der Xylanpolysulfatlösung getropft. Die entstehende Suspension enthält Teilchen, deren Durchmesser im 5 »m Bereich liegt. Zur Danuorubicinfreisetzung siehe Beispiel 12.

### Beispiel 8

### Polyelektrolytkomplexpartikel aus Palmitoylxylanpolysulfat mit 20 % Palmitinsäure, Daunorubicin und Lysinoctadecylester

1 ml einer Lösung, die Daunorubicin und Lysinoctadecylester zu jeweils 1 % enthält, wird auf pH 4 eingestellt. Eine 1 % Lösung von Palmitoylxylanpolysulfat, ebenfalls 1 ml, ebenfalls auf pH 4 eingestellt, wird zugetropft. Die entstehende Suspension ist durch Filtrieren nicht mehr auftrennbar. Die Partikel können durch Variation der Konzentration, der Rührgeschwindigkeit, der Tropfengeschwindigkeit und des Düsendurchmessers im Bereich von 100 nm bis 1 »m eingestellt werden (s. Tabelle 1).

**Tabelle 1**

| Partikelgröße | Konzentration | Rührgeschwindigkeit | Tropfengeschwindigkeit | Düsendurchmesser |
|---|---|---|---|---|
| 1 »m | 0,1 % | 300 min⁻¹ | 100 min⁻¹ | 0,5 mm |
| 10 »m | 0,5 % | 300 min⁻¹ | 100 min⁻¹ | 0,5 mm |
| 20 »m | 1 % | 300 min⁻¹ | 100 min⁻¹ | 0,5 mm |
| 100 nm | 0,1 % | 1000 min⁻¹ | 100 min⁻¹ | 0,5 mm |
| 20 nm | 0,1 % | 1000 min⁻¹ | 100 min⁻¹ | 0,2 mm |
| 80 »m | 1 % | 100 min⁻¹ | 100 min⁻¹ | 0,5 mm |
| 100 »m | 1 % | 100 min⁻¹ | 200 min⁻¹ | 0,5 mm |

### Beispiel 9

### Echogene injizierbare Polyelektrolythkomplexmikropartikel als Ultraschallkontrastmittel

Die Polyelektrolytkomplexpartikel werden wie folgt hergestellt:
Jeweils eine 1 %ige wäßrige Lösung bei pH 7 werden angesetzt aus Dextransulfat (M = 6000), bei welchem die Dextran-OH-Gruppen zu ca. 20 % mit Capronsäure verestert und die restlichen OH-Gruppen sulfatiert wurden ("hydrophob verestertes Dextransulfat", "Polysäure") und aus einem Copolymer von Poly-α,β-(2-dimethylaminoethyl)-D,L-aspartamid (60 %) und Poly-α,β-(2-[palmitoyloxy]-ethyl-D,L-aspartamid (40 %) ("Polybase"). Die Polysäurelösung wird zur Polybaselösung getropft, 10 Minuten bei Raumtemperatur gerührt, der Komplex abzentrifugiert, die Lösung abdekantiert, der Feststoff mit H₂O aufgeschlämmt und gefriergetrocknet.

Test als Kontrastmittel:
Gefriergetrocknete und resuspendierte Mikropartikel der Größenordnung 1-3 »m werden in einem Phantom, welches ein Modell des Herzens sowie kleinster Kapillargefäße (Lungenmodell) darstellt, auf durch eingeschlossene Luft hervorgerufenen Ultraschallkontrast untersucht. Die Partikel passieren die Kapillaren ungehindert.

### b) Freisetzungsversuche

### Beispiel 10

### Freisetzung von Albumin aus den unter Beispiel 3 beschriebenen Mikropartikeln

Eine Mikropartikelsuspension in Phosphatpuffer wird kontinuierlich geschüttelt. Nach 1,4,7,13,21,28 Tagen wird der Überstand abgenommen und dann über literaturbekannte Elektrophorese der Albumingehalt bestimmt. Es ergibt sich ein Profil mit 2 Freisetzungsmaxima, wie es für verschiedene Impfstoffe erforderlich ist:

| | | | | | | |
|---|---|---|---|---|---|---|
| Freisetzung am Tag | 1 | 4 | 7 | 13 | 21 | 28 |
| Albumin freigesetzt | 50 | 20 | <5 | <5 | 10 | <5 |

Nach etwas mehr als einem Monat sind keine Partikel mehr vorhanden.

### Beispiel 11

### Tetracyclinfreisetzung aus den Polyelektrolytkomplexpartikeln nach Beispiel 6

Der Abbauversuch wird wie Versuch 10 durchgeführt. Der Wirkstoff wird nach literaturbekannter Methode über UV-Spektroskopie bestimmt.

Es ergeben sich:

| | | | | |
|---|---|---|---|---|
| Freisetzung am Tag | 1 | 4 | 7 | 13 |
| Tetracyclin freigesetzt | 30 | 10 | 10 | <5 |

Nach 13 Tagen war kein Tetracyclin mehr nachweisbar.

### Beispiel 12

### Daunorubicinfreisetzung aus den Polyelektrolytpartikeln nach Beispiel 7

Die Partikelsuspension wird in einen Soxhlet-Extraktor gefüllt und über mehrere Tage mit H₂0 extrahiert. Im Extrakt wird Daunorubicin nach literaturbekannter Methode fluorometrisch bestimmt (bei 472/555 nm). Es ergibt sich, bezogen auf die eingewogene Gesamtmenge Daunorubicin, folgende Freisetzung:

| | | | | | |
|---|---|---|---|---|---|
| nach | 3.5h | 11h | 20h | 29h | waren freigesetzt: |
| | 8.5% | 10.5% | 15.0% | 26.2% | der eingesetzten |

Wirkstoffmenge.

## Patentansprüche

1. Pharmazeutische Zubereitung in mikropartikulärer Form enthaltend einen Polyelektrolytkomplex aus Polykationen mit Polyanionen und mindestens einen zusätzlichen Wirkstoff.

2. Pharmazeutische Zubereitung nach Anspruch 1, dadurch gekennzeichnet, daß der Wirkstoff ausgewählt wird aus: aktiven Peptiden, Proteinen, Enzymen, Enzyminhibitoren, Antigenen, Cytostatika, Antiinflammatorika, Antibiotika, Vakzinen.

3. Pharmazeutische Zubereitung nach Anspruch 1, dadurch gekennzeichnet, daß die Mikropartikel neben dem Wirkstoff Hilfsstoffe wie Adjuvantien oder amphiphile Molekülen enthalten.

4. Pharmazeutische Zubereitung nach Anspruch 1 bis 3, dadurch gekennzeichnet, daß der Wirkstoff eine ultraschallkontrastgebende Substanz ist.

5. Pharmazeutische Zubereitung nach einem oder mehreren der Ansprüche 1 bis 4, dadurch gekennzeichnet, daß der Polyelektrolytkomplex aus mindestens einer Substanz aufgebaut ist, die polymer ist.

6. Pharmazeutische Zubereitung nach Anspruch 5, dadurch gekennzeichnet, daß die Substanz aus mindestens zwei Monomereinheiten besteht.

7. Pharmazeutische Zubereitung nach einem oder mehreren der Ansprüche 1 bis 6, dadurch gekennzeichnet, daß der Polyelektrolytkomplex eine mittlere Teilchengröße von ≦5 »m aufweist.

8. Pharmazeutische Zubereitung nach einem oder mehreren der Ansprüche 1 bis 7, dadurch gekennzeichnet, daß der Polyelektrolytkomplex eine mittlere Teilchengröße von kleiner als 15 »m aufweist.

9. Pharmazeutische Zubereitung nach einem oder mehreren der Ansprüche 1 bis 8, dadurch gekennzeichnet, daß der Polyelektrolytkomplex eine mittlere Teilchengröße von kleiner als 100 »m aufweist.

10. Pharmazeutische Zubereitung nach einem oder mehreren der Ansprüche 1 bis 9, dadurch gekennzeichnet, daß der Polyelektrolytkomplex eine Polysäure enthält, die ausgewählt wird aus: Xylanpolysulfaten, Dextransulfaten, Polyaminosäuren wie Polyasparaginsäure oder Polyglutaminsäure, Polysaccharidpolysulfaten wie Sulfaten von Stärkehydrolysaten, Inulin, Hydroxyethylstärken, Polysaccharidpolysulfonaten, Polysaccharidpolyphosphaten, Polyphosphaten.

11. Pharmazeutische Zubereitung nach einem oder mehreren der Ansprüche 1 bis 9, dadurch gekennzeichnet, daß der Polyelktrolytkomplex eine Polysäure enthält, die ausgewählt wird aus:
Jeweils teilweise hydrophobisierten (z.B. verethert, verestert) Derivaten von Xylanpolysulfat, Polysulfaten anderer Polyzucker wie z.B. Stärkehydrolysaten, Inulin, Hydroxyethylstärken, Dextranen;
von Polyaminosäuren wie Polyasparaginsäure oder Polyglutaminsäure sowie von Polysaccharidpolysulfonaten, Polysaccharidpolyphosphonaten, Polyphosphaten.

12. Pharmazeutische Zubereitung nach einem oder mehreren der Ansprüche 1 bis 11, dadurch gekennzeichnet, daß der Polyelektrolytkomplex eine Polybase enthält, die ausgewählt wird aus: Poly-L-Lysin, Poly-α,β-(2-dimethylamino ethyl)-D,L-aspartamid, Chitosan, Lysinoctadecylester, aminierten Dextranen, aminierten Cyclodextrinen, aminierten Celluloseethern, aminierten Pektinen.

13. Pharmazeutische Zubereitung nach einem oder mehreren der Ansprüche 1 bis 11, dadurch gekennzeichnet, daß der Polyelektrolytkomplex eine Polybase enthält, die ausgewählt wird aus:
jeweils (z.B. durch teilweise oder vollständige Veresterung und/oder Veretherung) hydrophobisierte Derivate von: Poly-L-Lysin verschiedener Molekulargewichtsbereiche, Poly-α,β-(2-dimethylamino - ethyl)-D,L-aspartamid, Chitosan, aminierten Dextranen, aminierten Cyclodextrinen, aminierten Celluloseethern, aminierten Pektinen sowie Copolymere aus Poly-α,β-(2-dimethylaminoethyl)-D,L-aspartamid und hydrophob verestertem Poly-α,β-(2-hydroxyethyl)-D,L-aspartamid.

14. Verfahren zur Herstellung einer pharmazeutischen Zubereitung gemäß Anspruch 1, dadurch gekennzeichnet, daß man eine Lösung von einer sauren und einer basischen Substanz zusammenbringt, wobei mindestens eine der Substanzen polymer ist und wobei
a) eine der Substanzen einen Wirkstoff in chemisch gebundener Form enthält oder
b) der Wirkstoff in der Lösung der sauren oder in der Lösung der basischen Substanz enthalten ist
und anschließend der entstandene Polyelektrolytkomplex in mikropartikulärer Form entsteht oder gegebenenfalls in eine mikropartikuläre Form überführt wird.

## Claims

1. A pharmaceutical composition in microparticulate form containing a polyelectrolyte complex of polycations with polyanions and at least one additional active substance.

2. A pharmaceutical composition as claimed in claim 1, wherein the active substance is selected from: active peptides, proteins, enzymes, enzyme inhibitore, antigens, cytostatics, antiinflammatory agents, antibiotics and vaccines.

3. A pharmaceutical composition as claimed in claim 1, wherein the microparticles contain auxiliaries such as adjuvants or amphiphilic molecules besides the active substance.

4. A pharmaceutical composition as claimed in claim 1 to 3, wherein the active substance is an ultrasonic constrast substance.

5. A pharmaceutical composition as claimed in one or more of claims 1 to 4, wherein the polyelectrolyte complex is composed of at least one substance which is polymeric.

6. A pharmaceutical composition as claimed in claim 5, wherein the substance is composed of at least two monomer units.

7. A pharmaceutical composition as claimed in one or more of claims 1 to 6, wherein the polyelectrolyte complex has an average particle size of ≦5 »m.

8. A pharmaceutical composition as claimed in one or more of claims 1 to 7, wherein the polyelectrolyte complex has an average particle size of less than 15 »m.

9. A pharmaceutical composition as claimed in one or more of claims 1 to 8, wherein the polyelectrolyte complex has an average particle size of less than 100 »m.

10. A pharmaceutical composition as claimed in one or more of claims 1 to 9, wherein the polyelectrolyte complex contains a polyacid which is selected from: xylan polysulfates, dextran sulfates, poly(amino acids) such as polyaspartic acid or polyglutamic acid, polysaccharide polysulfates such as sulfates of starch hydrolysates, inulin, hydroxyethylstarches, polysaccharide polysulfonates, polysaccharide polyphosphates, polyphosphates.

11. A pharmaceutical composition as claimed in one or more of claims 1 to 9, wherein the polyelectrolyte complex contains a polyacid which is selected from: in each case partially hydrophobized (for example etherified, esterified) derivatives of xylan polysulfate, polysulfates of other polysaccharides such as, for example, starch hydrolysates, inulin, hydroxyethylstarches, dextrans; of poly(amino acids) such as polyaspartic acid or polyglutamic acid, and of polysaccharide polysulfonates, polysaccharide polyphosphonates, polyphosphates.

12. A pharmaceutical composition as claimed in one or more of claims 1 to 11, wherein the polyelectrolyte complex contains a polybase which is selected from: poly-L-lysine, poly-α,β-(2-dimethylaminoethyl)-D,L-aspartamide, chitosan, lysine octadecyl ester, aminated dextrans, aminated cyclodextrins, aminated cellulose ethers, aminated pectins.

13. A pharmaceutical composition as claimed in one or more of claims 1 to 11, wherein the polyelectrolyte complex contains a polybase which is selected from: in each case (for example by partial or complete esterification and/or etherification) hydrophobized derivatives of: poly-L-lysine of various molecular weight ranges, poly-α,β-(2-dimethylaminoethyl)-D,L-aspartamide, chitosan, aminated dextrans, aminated cyclodextrins, aminated cellulose ethers, aminated pectins and copolymers of poly-α,β-(2-dimethylaminoethyl)-D,L-aspartamide and hydrophobically esterified poly-α,β-(2-hydroxyethyl)-D,L-aspartamide.

14. A process for preparing a pharmaceutical composition as claimed in claim 1, which comprises mixing a solution of an acidic and of a basic substance, where at least one of the substances is polymeric and where
a) one of the substances contains an active substance in chemically bound form, or
b) the active substance is contained in the solution of the acidic or in the solution of the basic substance,
and subsequently the resulting polyelectrolyte complex is produced in microparticulate form or, where appropriate, is converted into a microparticulate form.

## Revendications

1. Composition pharmaceutique sous forme microparticulaire, contenant un complexe de polyélectrolytes constitué de polycations avec des polyanions, et au moins une substance active supplémentaire.

2. Composition pharmaceutique selon la revendication 1, caractérisée en ce que la substance active est choisie parmi des peptides actifs, des protéines actives, des enzymes, des inhibiteurs d'enzyme, des antigènes, des agents cytostatiques, des anti-inflammatoires, des antibiotiques, des vaccins.

3. Composition pharmaceutique selon la revendication 1, caractérisée en ce que les microparticules contiennent, en plus de la substance active, des substances auxiliaires telles que des adjuvants ou des molécules amphiphiles.

4. Composition pharmaceutique selon les revendications 1 à 3, caractérisée en ce que la substance active est une substance de contraste ultrasonique.

5. Composition pharmaceutique selon une ou plusieurs des revendications 1 à 4, caractérisée en ce que le complexe de polyélectrolytes est constitué d'au moins une substance qui est polymère.

6. Composition pharmaceutique selon la revendication 5, caractérisée en ce que la substance est constituée d'au moins deux motifs monomères.

7. Composition pharmaceutique selon une ou plusieurs des revendications 1 à 6, caractérisée en ce que le complexe de polyélectrolytes présente une taille moyenne de particules ≦5 »m.

8. Composition pharmaceutique selon une ou plusieurs des revendications 1 à 7, caractérisée en ce que le complexe de polyélectrolytes présente une taille moyenne de particules <15 »m.

9. Composition pharmaceutique selon une ou plusieurs des revendications 1 à 8, caractérisée en ce que le complexe de polyélectrolytes présente une taille moyenne de particules <100 »m.

10. Composition pharmaceutique selon une ou plusieurs des revendications 1 à 9, caractérisée en ce que le complexe de polyélectrolytes contient un polyacide qui est choisi parmi des polysulfates de xylane, des sulfates de dextran, des polyaminoacides tels que le poly(acide aspartique) ou le poly(acide glutamique), des polysulfates de polysaccharides, tels que des sulfates d'hydrolysats d'amidon, l'inuline, des hydroxyéthylamidons, des polysulfonates de polysaccharides, des polyphosphates de polysaccharides, des polyphosphates.

11. Composition pharmaceutique selon une ou plusieurs des revendications 1 à 9, caractérisée en ce que le complexe de polyélectrolytes contient un polyacide qui est choisi parmi: des dérivés dans chaque cas partiellement rendus hydrophobes (par exemple éthérifiés, estérifiés) de polysulfate de xylane, de polysulfates d'autres polysaccharides comme par exemple des hydrolysats d'amidon, l'inuline, des hydroxyéthylamidons, des dextrans; de polyaminoacides tels que le poly(acide aspartique) ou le poly(acide glutamique) ainsi que de polysulfonates de polysaccharides, de polyphosphonates de polysaccharides, de polyphosphates.

12. Composition pharmaceutique selon une ou plusieurs des revendications 1 à 11, caractérisée en ce que le complexe de polyélectrolytes contient une polybase qui est choisie parmi: la poly-L-lysine, le poly-α,β-(2-diméthylaminoéthyl)-D,L-aspartamide, le chitosane, l'ester octadécylique de lysine, des dextrans aminés, des cyclodextrines aminées, des éthers de cellulose aminés, des pectines aminées.

13. Composition pharmaceutique selon une ou plusieurs des revendications 1 à 11, caractérisée en ce que le complexe de polyélectrolytes contient une polybase qui est choisie parmi: des dérivés rendus chacun hydrophobes (par exemple par estérification et/ou éthérification partielle ou totale) de: poly-L-lysine à diverses plages de masse moléculaire, poly-α,β-(2-diméthylaminoéthyl)-D,L-aspartamide, chitosane, dextrans aminés, cyolodextrines aminées, éthers de cellulose aminés, pectines aminées ainsi que oopolymères de poly-α,β-(2-diméthylaminoéthyl)-D,L-aspartamide et de poly-α,β-(2-hydroxyéthyl)-D,L-aspartamide estérifiés pour être rendus hydrophobes.

14. Procédé pour la préparation d'une composition pharmaceutique selon la revendication 1, caractérisé en ce que l'on met en contact une solution d'une substance acide et une solution d'une substance basique, au moins l'une des substances étant polymère, et
a) l'une des substances contenant une substance active, sous forme liée chimiquement, ou
b) la substance active étant contenue dans la solution de la substance acide ou dans la solution de la substance basique,
et le complexe de polyélectrolytes résultant apparaissant sous forme microparticulaire ou étant ensuite éventuellement converti en une forme microparticulaire.
